(19) Europäisches Patentamt
European Patent Office
Office européen des brevets

(11) Veröffentlichungsnummer: **0 012 973**
**B1**

# (12) EUROPÄISCHE PATENTSCHRIFT

(45) Veröffentlichungstag der Patentschrift: **15.07.81**

(51) Int. Cl.³: **C 07 C 127/24,** C 08 G 18/78, C 08 G 18/80

(21) Anmeldenummer: **79105223.6**

(22) Anmeldetag: **17.12.79**

## (54) Verfahren zur Herstellung von Biuret- und/oder höhere Polyuretgruppen aufweisenden Polyisocyanaten, die nach diesem Verfahren erhältlichen Verbindungen, sowie ihre Verwendung als Aufbaukomponente bei der Herstellung von Polyurethankunststoffen.

(30) Priorität: **30.12.78 DE 2856826**

(43) Veröffentlichungstag der Anmeldung: **09.07.80 Patentblatt 80/14**

(45) Bekanntmachung des Hinweises auf die Patenterteilung: **15.07.81 Patentblatt 81/28**

(84) Benannte Vertragsstaaten: **BE DE FR GB IT NL**

(56) Entgegenhaltungen: **EP-A-0 000 194**

(73) Patentinhaber: **BAYER AG, Zentralbereich Patente, Marken und Lizenzen, D-5090 Leverkusen 1, Bayerwerk (DE)**

(72) Erfinder: **Reichmann, Wolfgang, Dr., Vohwinkelallee 19, D-4000 Düsseldorf 1 (DE)**
Erfinder: **König, Klaus, Dr., Heymannstrasse 50, D-5090 Leverkusen 1 (DE)**
Erfinder: **Schönfelder, Manfred, Dr., Höhenstrasse 126, D-5090 Leverkusen 3 (DE)**

EP 0 012 973 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Biuret- und/oder höhere Polyuretgruppen aufweisenden Polyisocyanaten, die nach diesem Verfahren erhältlichen Verbindungen, sowie ihre Verwendung als Aufbaukomponente bei der Herstellung von Polyurethankunststoffen

Die Erfindung betrifft ein neues Verfahren zur Herstellung von Biuret- und/oder Polyuretgruppen aufweisenden Polyisocyanaten, die nach diesem Verfahren zugänglichen Polyisocyanate und die Verwendung der Verfahrensprodukte als Isocyanat-Komponente bei der Herstellung von Polyurethankunststoffen.

Biuretgruppen enthaltende Polyisocyanate sind bekannt und finden als Rohstoffe für hochwertige, lichtechte Lackierungen praktische Verwendung. Sie können beispielsweise aus Diisocyanaten und Wasser (DE-AS 1 101 394), Schwefelwasserstoff (DE-AS 1 165 580), Ameisensäure (DE-AS 1 174 760), tertiären Alkoholen (DE-AS 1 543 178, DE-AS 1 931 055) oder Monoaminen (De-OS 2 308 015) hergestellt werden.

Bei diesem Verfahren des Standes der Technik entstehen aus einem Teil der Isocyanatgruppen zunächst Aminogruppen, die mit überschüssigem Diisocyanat über die entsprechenden Harnstoffdiisocyanate zu Biuretpolyisocyanaten weiterreagieren. Die Umwandlung der Isocyanatgruppen in Aminogruppen wird stets durch die Entstehung von gasförmigen Nebenprodukten wie Kohlendioxid, Kohlenmonoxid, Kohlensulfoxid oder Olefine begleitet, deren Beseitigung zu Abgasproblemen führen kann. Bei der heterogenen Reaktion von Diisocyanaten mit Wasser besteht zusätzlich die Gefahr der Bildung von unlöslichen Polyharnstoffen, die nur schwer abzutrennen sind. Von besonderem Nachteil ist jedoch der Umstand, dass bei diesen Verfahren zunächst ein Teil der Isocyanatgruppen des als Ausgangsmaterial verwendeten Diisocyanate unter Aminbildung vernichtet wird.

Es mangelte daher auch nicht an Versuchen, Biuretgruppen aufweisende Polyisocyanate durch direkte Umsetzung von Diaminen mit Diisocyanaten ohne Abspaltung von flüchtigen Nebenprodukten und ohne Vernichtung von Isocyanatgruppen für Aminbildung herzustellen.

Wegen der hohen Reaktivität von aliphatischen Aminogruppen gegenüber Isocyanatgruppen traten insbesondere bei Umsetzung von primären Diaminen mit Diisocyanaten erhebliche praktische Schwierigkeiten auf, da die Tendenz zur Bildung von unlöslichen Polyharnstoffen und vernetzten Produkten sehr gross ist.

Wie aus der DE-OS 2 261 065 beispielsweise hervorgeht, ist zur Vervollständigung der Umsetzung bei technisch leicht zugänglichen Ausgangsmaterialien wie Hexamethylendiamin und Hexamethylendiisocyanat unwirtschaftlich langes Nachheizen bei hoher Temperatur notwendig. Dies führt zu einer starken Beeinträchtigung der Eigenschaften der Verfahrensprodukte, insbesondere deren Eigenfarbe. Diese Nachteile können nach dem Verfahren der DE-OS 2 609 995 beseitigt werden, wenn das Diamin unter besonderen Vorkehrungen dampfförmig in das Diisocyanat eingeleitet wird. Bei diesem Verfahren muss jedoch sehr darauf geachtet werden, dass kein Diisocyanat in das Einleitungsrohr gelangt, weil sonst schnell durch Harnstoffbildung verursachte Verstopfungen auftreten können.

Bei diesen Verfahren des Standes der Technik finden während der Bildung der Biuretpolyisocyanate dauernde Umgruppierungsreaktionen statt, durch die in Abhängigkeit vom $NCO/NH_2$-Verhältnis das eingesetzte Diamin in das entsprechende Diisocyanat übergeht. Dadurch fallen bei der Abtrennung des nicht umgesetzten Diisocyanats vom Biuretpolyisocyanat als Destillate Gemische verschiedener Diisocyanate an, falls nicht Diamine und Diisocyanate gleicher Konstitution verwendet werden.

Weiterhin nachteilig hierbei ist, dass im Biuretpolyisocyanat selbst mehr oder weniger grosse Mengen des sich durch Umbiuretisierung aus dem Diamin bildenden Diisocyanats als Monomerenanteil zurückbleiben.

Gemäss der DE-OS 2 010 887 ist die direkte Umsetzung von sekundären Diaminen mit Diisocyanaten durchführbar. Nach dem dort beschriebenen Verfahren sind in erster Linie Biuretpolyisocyanate mit aromatisch gebundenen Isocyanatgruppen herstellbar, die jedoch für hochwertige und lichtechte Lackierungen ungeeignet sind. Im Falle der Verwendung von aliphatischen Diaminen in Kombination mit aliphatischen Diisocyanaten entstehen beim Verfahren der genannten Vorveröffentlichung zwar spontan die entsprechenden Bis-Harnstoff-diisocyanate, die jedoch nicht ohne weiteres mit weiterem Diisocyanat zu höherfunktionellen Biuretpolyisocyanaten umsetzbar sind.

Es war daher die Aufgabe der vorliegenden Erfindung, ein neues Verfahren zur Verfügung zu stellen, welches auf einfache Weise die Herstellung von hochwertigen modifizierten aliphatischen Polyisocyanaten gestattet, die die Vorteile der bekannten Biuretpolyisocyanate in sich vereinigen, ohne dass das Verfahren mit den genannten Nachteilen der Verfahren des Standes der Technik behaftet ist.

Diese Aufgabe konnte durch das nachstehend beschriebene erfindungsgemässe Verfahren gelöst werden.

Gegenstand der vorliegenden Erfindung ist ein Verfahren zur Herstellung von Biuret- und/oder höhere Polyuretgruppen aufweisenden Polyisocyanaten durch Umsetzung von sekundären Diaminen der Formel

$$R_1\text{-NH-}R_2\text{-NH-}R_3$$

mit überschüssigen Mengen an organischen Diisocyanaten der Formel

$$R_4(NCO)_2$$

wobei

$R_1$ und $R_3$ für gleiche oder verschiedene Reste stehen und aliphatische Kohlenwasserstoffreste

mit 1 bis 20 Kohlenstoffatomen, die auch miteinander verknüpft unter Einbeziehung des Diamingrundgerüstes einen 5- oder 6gliedrigen Ring bilden können, oder cycloaliphatische Kohlenwasserstoffreste mit 4 bis 15 Kohlenstoffatomen bedeuten, und

$R_2$ und $R_4$ für gleiche oder verschiedene Reste stehen und aliphatische, gegebenenfalls Estergruppen aufweisende Kohlenwasserstoffreste mit insgesamt 2 bis 20 Kohlenstoffatomen oder cycloaliphatische Kohlenwasserstoffreste mit 4 bis 15 Kohlenstoffatomen bedeuten, wobei jeweils zwischen den beiden Stickstoffatomen mindestens 2 Kohlenstoffatome angeordnet sind,

dadurch gekennzeichnet, dass man die Umsetzung in Gegenwart von katalytischen Mengen an starken, mit Isocyanaten gemischte Carbamidsäureanhydride bildenden Säuren durchführt.

Gegenstand der vorliegenden Erfindung ist auch eine Abänderung dieses Verfahrens, welche dadurch gekennzeichnet ist, dass man in einem ersten Reaktionsschritt die bei dem obengenannten Verfahren einzusetzenden Diamine und Diisocyanate zunächst in Abwesenheit eines Katalysators zu einem Harnstoffdiisocyanat der Formel

$$OCN-R_4-NH-CO-\overset{R_1}{\overset{|}{N}}-R_2-\overset{R_3}{\overset{|}{N}}-CO-NH-R_4-NCO$$

umsetzt, und man anschliessend dieses Harnstoffdiisocyanat in Gegenwart der obengenannten Katalysatoren mit weiterem Diisocyanat der Formel

$R_4(NCO)_2$

oder mit einem anderen Diisocyanat der Formel

$R_5(NCO)_2$

zur Reaktion bringt, wobei $R_1$, $R_2$, $R_3$ und $R_4$ die obengenannte Bedeutung haben und $R_5$ der Definition von $R_4$ entspricht, mit $R_4$ jedoch nicht identisch ist.

Gegenstand der vorliegenden Erfindung sind auch die nach diesem Verfahren erhältlichen Biuret- und/oder höhere Polyuretgruppen aufweisenden Polyisocyanate.

Gegenstand der vorliegenden Erfindung ist schliesslich auch die Verwendung der nach diesem Verfahren erhältlichen Biuret- und/oder höhere Polyuretgruppen aufweisenden Polyisocyanate, gegebenenfalls in mit Blockierungsmittel für Isocyanatgruppen blockierter Form als Aufbaukomponente bei der Herstellung von Polyurethankunststoffen nach dem Isocyanat-Polyadditionsverfahren.

In obigen Formeln und auch nachstehend haben die Reste $R_1$, $R_2$, $R_3$, $R_4$ und $R_5$ die obengenannte Bedeutung. Die bevorzugte Bedeutung dieser Reste ist die folgende:

$R_1$ und $R_3$ stehen für gleiche oder verschiedene Reste und bedeuten vorzugsweise jeweils aliphatische Kohlenwasserstoffreste mit 1 bis 3 Kohlenstoffatomen,

$R_2$ steht vorzugsweise für einen aliphatischen Kohlenwasserstoffrest mit 2 bis 6 Kohlenstoffatomen oder einen cycloaliphatischen Kohlenwasserstoffrest mit 6 bis 10 Kohlenwasserstoffatomen,

$R_4$ und $R_5$ stehen für verschiedene Kohlenwasserstoffreste und bedeuten vorzugsweise aliphatische Kohlenwasserstoffreste mit 6 bis 10 Kohlenstoffatomen oder cycloaliphatische Kohlenwasserstoffreste mit 6 bis 10 Kohlenstoffatomen. $R_4$ steht besonders bevorzugt für einen Hexamethylenrest.

Ausgangsmaterialien für das erfindungsgemässe Verfahren sind Diamine der Formel

$R_1-NH-R_2-NH-R_3$

und Diisocyanate der Formel

$R_3(NCO)_2$

und gegebenenfalls der Formel

$R_5(NCO)_2$.

Beispiele geeigneter Diamine der genannten Formel sind N,N′-Dimethyl-äthylendiamin, N,N′-Diäthyl-äthylendiamin, N,N′-Diisopropyl-äthylendiamin, N,N′-Diisopropyl-trimethylendiamin, N,N′-Diisopropyl-hexamethylendiamin, N-Methyl-N′-decyl-hexamethylendiamin, N-Cyclohexyl-N′-stearyl-äthylendiamin, N-Methyl-N′-äthyl-phenylendiamin-(1,4), 2,6-Bis-(Methylamino)-hexancarbonsäure-(1)-ethylester oder 1,4-Piperazin. Beliebige Gemische der beispielhaft genannten Diamine können ebenfalls eingesetzt werden.

Beim erfindungsgemässen Verfahren einzusetzende Diisocyanate sind beispielsweise Tetramethylendiisocyanat, Hexamethylendiisocyanat, 2,4,4-Trimethyl-hexamethylendiisocyanat, Undecamethylendiisocyanat, 1-Isocyanato-3-isocyanatomethyl-3,5,5-trimethylcyclohexan (Isophorondiisocyanat), 1,4-Diisocyanatocyclohexan, 4,4′-Diisocyanato-dicyclohexylmethan, 1,2-Bis-(isocyanatomethyl)-cyclobutan oder 6-Isocyanatocapronsäure-2-isocyanatoäthylester.

Während die Reaktion von primären oder sekundären Diaminen mit Diisocyanaten zu den entsprechenden Harnstoffen, bzw. Biureten zum vorbekannten Stand der Technik gehört, ist bislang noch kein Verfahren bekannt geworden, welches bei Verwendung eines sekundären Diamins die Weiterreaktion der zwischen Amin und Isocyanat ablaufenden Additionsreaktion über die Biuretstufe hinaus gestattet. Dies liegt an der Reaktionsträgheit der Biurete gegenüber aliphatischen Diisocyanaten, die nur durch Verwendung geeigneter Katalysatoren überwunden werden kann. Bei Verwendung der erfindungsgemäss einzusetzenden Katalysatoren ist ohne weiteres eine Weiterreaktion der Biuretpolyisocyanate zu höheren Polyuretgruppen aufweisenden Polyisocyanaten möglich. Durch die erfindungsgemässen Katalysatoren wird darüber hinaus jedoch auch die bereits sehr träge Reaktion der aus den Diaminen und Diisocyanaten zunächst entstehenden Harnstoffdiisocyanate mit weiterem Diisocyanat erleichtert, so dass ein langzeitiges Erhitzen der Reaktionspartner auf hohe Temperaturen nicht erforderlich ist.

Bei den erfindungsgemäss einzusetzenden Katalysatoren handelt es sich um protonenabspaltende starke Säuren, welche mit Isocyanaten, insbesondere mit aliphatischen oder cycloaliphatischen Isocyanaten, unter Bildung eines gemischten Säureanhydrids reagieren, wobei die dem Isocyanat entsprechende Carbamidsäure und die protonenabspaltende Säure die Säuren des gemischten Säureanhydrids darstellen. So reagieren derartige, für das erfindungsgemässe Verfahren geeignete Säuren HX (X = Säurerest nach Abspaltung des Protons) mit Isocyanaten X-NCO zu Addukten der Formel Y-NH-CO-X, welche als gemischtes Anhydrid der Carbamidsäure Y-NH-COOH und der Säure HX anzusehen sind.

Beispiele geeigneter Säuren sind Halogenwasserstoffe wie z.B. Fluorwasserstoff, Chlorwasserstoff, Bromwasserstoff oder Jodwasserstoff, Chlorsulfonsäure, Fluorsulfonsäure, Schwefelsäure, Alkansulfonsäuren wie z.B. Methansulfonsäure oder perhalogenierte Alkansulfonsäure wie z.B. Trifluormethansulfonsäure. Chlorwasserstoff ist die beim erfindungsgemässen Verfahren bevorzugt einzusetzende Säure. Anstelle der Säuren können beim erfindungsgemässen Verfahren selbstverständlich sowohl die den Säuren entsprechenden Ammoniumsalze mit den als Ausgangsmaterial eingesetzten Aminen oder die den Säuren entsprechenden gemischten Carbamidsäureanhydride, insbesondere Carbamidsäurechloride, der als Ausgangsmaterial eingesetzten Diisocyanate oder eines beliebigen anderen Isocyanats eingesetzt werden. Im allgemeinen werden die Katalysatoren in Mengen von 0,001-10, vorzugsweise 0,01-1,0 Gew.-%, bezogen auf das Gesamtgewicht der Reaktionspartner, eingesetzt.

Die Durchführung des erfindungsgemässen Verfahrens erfolgt im allgemeinen innerhalb des Temperaturbereichs von 0 bis 140 °C, vorzugsweise 50 bis 90 °C, wobei die über die Stufe der Harnstoffdiisocyanate hinausführende Reaktion zu Biuret- und höheren Polyuretgruppen aufweisenden Polyisocyanaten im allgemeinen im Bereich zwischen 90 und 140 °C stattfindet. Die erfindungsgemässe Katalyse mit den beispielhaft genannten Säuren gestattet somit unter milden Reaktionsbedingungen die Herstellung von Biuret- und Polyuretgruppen aufweisenden Isocyanat-Additionsprodukten aus aliphatischen Diisocyanaten und aus aliphatischen sekundären Diaminen.

Die Durchführung des erfindungsgemässen Verfahrens erfolgt im wesentlichen nach zwei Varianten:

Gemäss einer ersten Ausführungsform des erfindungsgemässen Verfahrens erfolgt die Katalysatorzugabe bereits zu Beginn der Reaktion. Man kann dabei den Katalysator beispielsweise mit dem Diisocyanat vorlegen oder ihn als Ammoniumsalz mit den Aminen eindosieren. Das Diisocyanat und das Diamin werden somit bei dieser ersten Ausführungsform des erfindungsgemässen Verfahrens bereits von Anfang an in Gegenwart des Katalysators umgesetzt. Man legt hierzu vorzugsweise das Diisocyanat bei einer Temperatur von ca. 0 bis 100 °C vor und trägt das Diamin in das vorgelegte Diisocyanat ein. Die Mengenverhältnisse der Reaktionspartner werden dabei so gewählt, dass im Reaktionsgemisch ein NCO/NH-Äquivalentverhältnis von ca. 4:1 bis 30:1 vorliegt. Es bilden sich hierbei spontan die entsprechenden Harnstoffdiisocynate, während die Weiterreaktion zum Biuret- und/oder Polyuretgruppen aufweisenden Endprodukt im allgemeinen im Anschluss hieran durch einfaches Erhitzen auf ca. 90 bis 140 °C stattfindet. Der Reaktionsverlauf kann durch Kontrolle der Isocyanatgehaltabnahme verfolgt werden. Ein Abbruch der Reaktion ist dann durch einfaches Abkühlen auf Raumtemperatur möglich. Bei Verwendung von flüchtigen Katalysatoren kann zur Vermeidung von bei höheren Temperaturen möglichen Katalysatorverlusten unter Druck gearbeitet werden. Die benötigten Reaktionszeiten hängen von der Art der Ausgangsprodukte, von der Temperatur und insbesondere von der Art und Menge des Katalysators ab. Sie betragen im allgemeinen 1-20 vorzugsweise 2-8 Stunden. Nach Beendigung der Reaktion erhält man klare, farblose bis schwach gelblich gefärbte Reaktionslösungen. Dies ist insbesondere auf die relativ niedrige Reaktionstemperatur zurückzuführen.

Gemäss einer zweiten Ausführungsform des erfindungsgemässen Verfahrens wird zunächst aus einem Diamin und einem Diisocyanat der beispielhaft genannten Art in Abwesenheit eines Katalysators ein Harnstoffdiisocyanat der Formel

$$OCN-R_4-NH-CO-\underset{|}{\overset{R_1}{N}}-R_2-\underset{|}{\overset{R_3}{N}}-CO-NH-R_4-NCO$$

hergestellt. Auch hierbei kommen die Reaktionspartner in solchen Mengen zum Einsatz, die einem NCO/NH-Äquivalentverhältnis von 4:1 bis 30:1 entsprechen. Die Reaktionstemperatur liegt hierbei im allgemeinen zwischen 0 und 100 °C, vorzugsweise zwischen 50 und 90 °C. Nach der auch in Abwesenheit des Katalysators spontan ablaufenden Harnstoffbildung wird in einer zweiten Reaktionsstufe nach Zugabe des Katalysators und vorzugsweise unter Erhöhung der Reaktionstemperatur auf ca. 90-140 °C die Biuret- und/oder Polyuretbildung, wie oben beschrieben, eingeleitet. Diese Bildung der Biurete bzw. höheren Polyurete kann jedoch auch dergestalt erfolgen, dass das im Gemisch mit dem Harnstoffdiisocyanat noch vorliegende überschüssige Diisocyanat der Formel

$R_4(NCO)_2$

beispielsweise destillativ entfernt und durch ein anderes Diisocyanat der Formel

$R_5(NCO)_2$

ersetzt wird, bevor durch die Zugabe des Katalysators und die genannte Temperaturerhöhung die Ausbildung der Biuret- bzw. Polyuretstrukturen stattfindet.

Bei dieser stufenweisen Durchführung des erfindungsgemässen Verfahrens ist vorzugsweise darauf zu achten, dass das Harnstoffdiisocyanat und das mit diesem zum Biuret bzw. Polyuret weiterreagierende Diisocyanat in solchen Mengen vorliegen, die einem NCO/NH-Äquivalentverhältnis von etwa 2:1 bis 30:1 entsprechen. Die Kontrolle des Reaktionsverlaufs und der Abbruch der Reaktion erfolgt wie bei der Beschreibung der ersten Ausführungsform oben angegeben.

Bei beiden Ausführungsformen des erfindungsgemässen Verfahrens wird die Reaktion im allgemeinen zu einem Zeitpunkt beendet, bei welchem im Mittel, bezogen auf ein Mol Aminogruppen des Diamins, insgesamt ca. 2 bis 2,5 Mol NCO-Gruppen verbraucht sind. Es ist jedoch auch möglich, einen höheren «Polyuretisierungsgrad» zu erreichen, d.h. pro Mol Aminogruppen drei und mehr NCO-Gruppen des Diisocyanats umzusetzen. Allerdings nehmen die Viskositäten der Produkte dann schnell zu.

Der Katalysator wird im allgemeinen durch Andestillieren des Reaktionsgemisches im Vakuum entfernt. Bei Verwendung von Halogenwasserstoffen als Katalysatoren kann die Beseitigung, insbesondere bei kleineren Katalysatormengen auch durch Zugabe äquimolarer Mengen Propylenoxid erfolgen. Ferner ist es möglich, den Katalysator durch z.B. Dünnschichtverdampfung zu entfernen, falls das Rohisocyanat von überschüssigem Diisocyanat befreit wird. Das Destillat der Dünnschichtdestillation, das dann neben dem Diisocyanat den Katalysator enthält, kann als Ausgangsmaterial wiederverwendet werden.

Ist die Entfernung von überschüssigem Diisocyanat vorgesehen, so erfolgt sie meistens durch Dünnschichtverdampfung; sie kann jedoch auch durch Extraktion mit geeigneten Lösungsmitteln, wie z.B. Hexan, Heptan etc. erreicht werden.

Die Rohisocyanate können als solche verwendet werden. In den meisten Fällen werden sie jedoch vorzugsweise durch Dünnschichtverdampfung oder Extraktion von monomeren Isocyanatanteilen befreit. Die monomeren freien Produkte sind hellgelbe Öle oder auch feste Harze; der NCO-Gehalt beträgt 5–22 Gew.-%.

Das Verfahren eignet sich vorzüglich für die kontinuierliche Durchführung. In diesen Fällen werden z.B. mehrere Reaktionsgefässe in Form einer Kaskade hintereinander geschaltet. Im ersten Reaktionsgefäss werden die Ausgangsprodukte, d.h. das Diisocyanat und das Diamin bzw. das vorab hergestellte Harnstoffdiisocyanat bei ca. 80 °C vermischt und mit dem Katalysator versetzt. Die Katalysatorzugabe kann auch im zweiten Reaktionsgefäss bei 90–140 °C erfolgen. Im dritten und gegebenenfalls in weiteren Reaktionsgefässen findet dann bei 90–140 °C die Weiterreaktion zum Polyuretpolyisocyanat statt, wobei durch Steuerung der Temperatur und der Verweilzeit der angestrebte «Polyuretisierungsgrad» eingestellt wird. Überschüssiges Diisocyanat und der Katalysator werden z.B. über einen Schlangenrohrverdampfer kombiniert mit nachgeschaltetem Dünnschichtverdampfer entfernt. Die aus Diisocyanat und Katalysator bestehenden Destillate werden vereinigt, gewünschtenfalls vom Katalysator befreit und wieder in den Prozess zurückgeführt. Das Polyisocyanat wird als Rückstand der Dünnschichtdestillation gewonnen.

Bei der Durchführung des erfindungsgemässen Verfahrens können die Eigenschaften der erhaltenen modifizierten Polyisocyanate, insbesondere deren NCO-Funktionalität, NCO-Gehalt, sowie die Viskosität nicht nur durch Wahl der geeigneten Ausgangsmaterialien, sondern besonders einfach durch Einstellung des «Polyuretisierungsgrades», d.h. der Zahl der pro Aminogruppe umgesetzten NCO-Gruppen gesteuert werden.

Der Hauptvorteil des erfindungsgemässen Verfahrens gegenüber den bekannten Verfahren des Standes der Technik gemäss DE-OS 2 261 065 oder DE-OS 2 609 995 ist darin zu sehen, dass die Bildung von in überschüssigem Diisocyanat schwer löslichem Polyharnstoff praktisch unterbleibt bzw. dass keine verfahrenstechnisch schwierig zu handhabende Massnahmen zu deren Verhinderung erforderlich sind. Das erfindungsgemässe Verfahren gestattet die Herstellung von hochwertigen Lackpolyisocyanaten ohne die Anwendung von extrem hohen, d.h. oberhalb 140 °C liegenden Temperaturen, so dass stets Produkte mit einer geringen Eigengabe und einem geringen Anteil an unerwünschten höhermolekularen Nebenprodukten erhalten werden.

Die erfindungsgemässen Verfahrensprodukte können insbesondere als Isocyanatkomponente bei der Herstellung von Polyurethankunststoffen nach dem Isocyanat-Polyadditionsverfahren eingesetzt werden. Sie eignen sich sowohl zur Herstellung von Polyurethan-Schaumstoffen, als auch zur Herstellung von Elastomeren, Beschichtungen oder Verklebungen. Insbesondere bei Verwendung der erfindungsgemässen Verfahrensprodukte für das erstgenannte Einsatzgebiet erübrigt sich oft ein Abdestillieren des überschüssigen Diisocyanats nach Beendigung der erfindungsgemässen Umsetzung. Die monomerenfreien erfindungsgemässen Verfahrensprodukte stellen hervorragende Rohstoffe zur Herstellung hochwertiger, wetterfester und lichtechter Lackierungen dar.

Bezüglich der Verwendung der erfindungsgemässen Verfahrensprodukte als «Lackisocyanate» ist insbesondere ihre ausgezeichnete Verträglichkeit mit handelsüblichen Polyhydroxy-Polyacrylaten hervorzuheben. Ein weiterer Vorteil der erfindungsgemässen Verfahrensprodukte gegenüber bekannten Biuretpolyisocyanaten ist darin zu sehen, dass die auf aus sekundären Diaminen und Diisocyanaten hergestellten Harnstoffdiisocyanaten basierenden Produkte gegen Monomerenrückspaltung stabil sind, d.h. dass auch während der Lagerung bei erhöhter Temperatur (50°C) der Monomerengehalt der erfindungsgemässen Polyisocyanate nicht ansteigt. Die erfindungsgemässen Verfahrensprodukte können auch in mit Blockierungsmittel für Isocyanatgruppen blockierter Form zur Herstellung von Zweikomponenten-Polyurethanlacken zum Einsatz gelangen. Es werden hierbei die an sich bekannten Blockierungsmittel wie z.B. ε-Caprolactam, Malonsäurediäthylester oder Acetessigsäureäthylester verwendet. Die Überführung der Verfahrensprodukte in die entsprechenden blokkierten Polyisocyanate erfolgt nach den längst bekannten Verfahren des Standes der Technik.

Die nachfolgenden Beispiele dienen der näheren Erläuterung der vorliegenden Erfindung. Alle Prozentangaben stellen, sofern nicht anderslautend vermerkt, Gewichtsprozente dar.

Beispiel 1

In einem 4 l-Vierhalskolben mit Rührer, Rückflusskühler und Kontaktthermometer wurden zu 3696 g (22 Mol) 1,6-Diisocyanatohexan 200 g (1 Mol) 1,6-Diisopropylaminohexan bei 80–90°C innerhalb von 1 Stunde zugetropft. Nach der Aminzugabe betrug der NCO-Gehalt der klaren Reaktionsmischung 45,3%. Dies entspricht einem Gesamtumsatz von 2 NCO-Gruppen, d.h. es hatte sich das entsprechende Harnstoffdiisocyanat gebildet. Nun wurde die Lösung mit 4 g Chlorwasserstoff versetzt und die Reaktionstemperatur auf 100–110°C erhöht. Nach 3 Stunden war der NCO-Gehalt der Lösung auf 43,2% gesunken (entsprechend einem Verbrauch von 4 NCO-Gruppen pro Mol Diamin). Die Reaktionslösung wurde auf Raumtemperatur abgekühlt. Die nachfolgende Dünnschichtdestillation ergab 750 g eines Polyisocyanats mit einem NCO-Gehalt von 17,8% und einer Viskosität von 130000 mPa·s bei 20°C (Restgehalt an monomeren 1,6-Diisocyanatohexan = 0,51%).

Beispiel 2

Analog Beispiel 1 wurden zu 3696 g (22 Mol) 1,6-Diisocyanatohexan 172 g (1 Mol) 1,4-Diisopropylaminobutan bei 70–80°C innerhalb von 20 Minuten zugetropft. Der NCO-Gehalt der klaren Reaktionslösung betrug 45,6% (entsprechend einem Verbrauch von 2 NCO-Gruppen). Nun wurde die Reaktionstemperatur auf 100–110°C erhöht und die Mischung mit 4 g Chlorwasserstoff versetzt. Nach 2 Stunden war der NCO-Gehalt der Lösung auf 43,4% gesunken (entsprechend einem Verbrauch von 4 NCO-Gruppen pro Mol Diamin). Die Reaktionslösung wurde wie im Beispiel 1 beschrieben aufgearbeitet. Es wurden 730 g eines Polyisocyanats mit einem NCO-Gehalt von 18,5% und einer Viskosität von 146000 mPa·s bei 20°C erhalten. Der Monomerenrestgehalt betrug 0,64%.

Beispiel 3

Analog Beispiel 1 wurden in einem 6 l-Vierhalskolben zu 4032 g (24 Mol) 1,6-Diisocyanatohexan, die 4 g Chlorwasserstoff enthielten, 172 g (1 Mol) 1,4-Diisopropylaminobutan bei 70–80°C innerhalb von 20 Min. getropft. Nach der Aminzugabe wurde die Reaktionstemperatur der klaren Lösung auf 100°C erhöht. Nach 8 Stunden Rühren bei dieser Temperatur betrug der NCO-Gehalt der Mischung 41,8% (entsprechend einem Verbrauch von 6,1 NCO-Gruppen pro Mol Diamin). Nach der Dünnschichtdestillation wurden 1056 g eines Polyisocyanats mit einem NCO-Gehalt von 20,1% und einer Viskosität von 24000 mPa·s bei 20°C erhalten (Monomerengehalt 0,49%).

Beispiel 4

Analog Beispiel 1 wurden in einem 6 l-Kolben zu 4032 g (24 Mol) 1,6-Diisocyanatohexan, die 4 g Chlorwasserstoff enthielten, 200 g (1 Mol) 1,6-Diisopropylaminohexan bei 90–100°C innerhalb von 30 Min. getropft. Nach 8 Stunden bei 110–115°C betrug der NCO-Gehalt der klaren Reaktionslösung 42,5% (entsprechend einem Verbrauch von 5,2 NCO-Gruppen pro Mol Diamin). Nach der Aufarbeit fielen 900 g eines Polyisocyanats mit einem NCO-Gehalt von 18,7% und einer Viskosität von 26100 mPa·s bei 20°C an (Monomerengehalt 0,35%).

**Patentansprüche**

1. Verfahren zur Herstellung von Biuret- und/oder höhere Polyuretgruppen aufweisenden Polyisocyanaten durch Umsetzung von sekundären Diaminen der Formel

$R_1-NH-R_2-NH-R_3$

mit überschüssigen Mengen an organischen Diisocyanaten der Formel

$R_4(NCO)_2$

wobei

$R_1$ und $R_3$ für gleiche oder verschiedene Reste stehen und aliphatische Kohlenwasserstoffreste mit 1 bis 20 Kohlenstoffatomen, die auch miteinander verknüpft unter Einbeziehung des Diamingrundgerüstes einen 5- oder 6gliedrigen Ring bilden können, oder cycloaliphatische Kohlenwasserstoffreste mit 4 bis 15 Kohlenstoffatomen bedeuten, und

$R_2$ und $R_4$ für gleiche oder verschiedene Reste stehen und aliphatische, gegebenenfalls Estergruppen aufweisende Kohlenwasserstoffreste mit insgesamt 2 bis 20 Kohlenstoffatomen oder cycloaliphatische Kohlenwasserstoffreste mit 4 bis 15 Kohlenstoffatomen bedeuten, wobei jeweils zwischen den beiden Stickstoffatomen mindestens 2 Kohlenstoffatome angeordnet sind,

dadurch gekennzeichnet, dass man die Umsetzung in Gegenwart von katalytischen Mengen an starken, mit Isocyanaten gemischte Carbamidsäureanhydride bildenden Säuren durchführt.

2. Abänderung des Verfahrens gemäss Anspruch 1, dadurch gekennzeichnet, dass man die in Anspruch 1 genannten Diamine zunächst in Abwesenheit der Katalysatoren mit den in Anspruch 1 genannten Diisocyanaten zu Harnstoffdiisocyanaten der Formel

$$OCN-R_4-NH-CO-\overset{R_1}{\overset{|}{N}}-R_2-\overset{R_3}{\overset{|}{N}}-CO-NH-R_4-NCO$$

umsetzt, und anschliessend dieses Harnstoffdiisocyanat in Gegenwart der in Anspruch 1 genannten Katalysatoren mit weiterem Diisocyanat der Formel

$R_4(NCO)_2$

oder einem weiteren Diisocyanat der Formel

$R_5(NCO)_2$

umsetzt, wobei

$R_1$, $R_2$, $R_3$ und $R_4$ die in Anspruch 1 genannte Bedeutung haben und

$R_5$ der Definition für $R_4$ entspricht, mit $R_4$ jedoch nicht identisch ist.

3. Gemäss Anspruch 1 und 2 erhältliche Biuret- und/oder höhere Polyuretgruppen aufweisende Polyisocyanate.

4. Verwendung der gemäss Anspruch 1 und 2 erhältlichen Biuret- und/oder höhere Polyuretgruppen aufweisenden Polyisocyanate, gegebenenfalls in mit Blockierungsmittel für Isocyanatgruppen blockierter Form als Aufbaukomponente bei der Herstellung von Polyurethankunststoffen nach dem Isocyanat-Polyadditionsverfahren.

**Claims**

1. A process for the preparation of polyisocyanates containing biuret and/or higher polyuret groups by reacting secondary diamines of the formula:

$R_1-NH-R_2-NH-R_3$

with excess quantities of organic diisocyanates of the formula:

$R_4(NCO)_2$

wherein

$R_1$ and $R_3$ represent equal or different radicals and denote aliphatic hydrocarbon radicals containing from 1 to 20 carbon atoms which may also be attached to one another with incorporation of the basic diamine skeleton to form a 5- or 6-membered ring, or cycloaliphatic hydrocarbon radicals containing from 4 to 15 carbon atoms; and

$R_2$ and $R_4$ represent equal or different radicals and denote aliphatic hydrocarbon radicals having a total of from 2 to 20 carbon atoms optionally containing ester groups or cycloaliphatic hydrocarbon radicals containing from 4 to 15 carbon atoms, at least 2 carbon atoms in each case being arranged between the two nitrogen atoms;

characterised in that the reaction is carried out in the presence of catalytic quantities of strong acids which form mixed carbamic acid anhydrides with isocyanates.

2. A modification of the process according to Claim 1, characterised in that the diamines mentioned in Claim 1 are initially reacted with the diisocyanates mentioned in Claim 1 in the absence of the catalysts to form urea diisocyanates of the formula:

$$OCN-R_4-NH-CO-\overset{R_1}{\overset{|}{N}}-R_2-\overset{R_3}{\overset{|}{N}}-CO-NH-R_4-NCO$$

and then this urea diisocyanate is reacted in the presence of the catalysts mentioned in Claim 1 with more diisocyanate of the formula:

$R_4(NCO)_2$

or with another diisocyanate of the formula:

$R_5(NCO)_2$

wherein

$R_1$, $R_2$, $R_3$ and $R_4$ have the meaning mentioned in Claim 1 and

$R_5$ corresponds to the definition of $R_4$, but is not identical to $R_4$.

3. Polyisocyanates containing biuret and/or higher polyuret groups obtainable according to Claims 1 and 2.

4. The use of the polyisocyanates containing biuret and/or higher polyuret groups obtainable according to Claims 1 and 2, optionally blocked with blocking agents for isocyanate groups, as synthesis component in the preparation of polyurethane plastics by the isocyanate-polyaddition process.

**Revendications**

1. Procédé pour la préparation de polyisocyanates présentant des groupes biuret et/ou des groupes polyuret supérieurs par réaction de diamines secondaires de formule

$R_1-NH-R_2-NH-R_3$

avec un excès de diisocyanates organiques de formule

$R_4(NCO)_3$

dans lesquelles

$R_1$ et $R_3$ sont des restes égaux ou différents et représentent des restes d'hydrocarbures aliphatiques ayant 1 à 20 atomes de carbone, qui, reliés entre eux, peuvent aussi former, avec intervention du squelette de base de la diamine, un noyau

à 5 ou 6 chaînons, ou bien des restes d'hydrocarbures cycloaliphatiques ayant 4 à 15 atomes de carbone; et

$R_2$ et $R_4$ sont des restes égaux ou différents et représentent des restes d'hydrocarbures aliphatiques ayant au total 2 à 20 atomes de carbone ou des restes d'hydrocarbures cycloaliphatiques ayant 4 à 15 atomes de carbone, présentant éventuellement des groupes ester, les deux atomes d'azote étant chaque fois séparés par au moins deux atomes de carbone, caractérisé en ce que l'on effectue la réaction en présence de quantités catalytiques d'acides forts formant avec des isocyanates des anhydrides mixtes d'acides carbamiques.

3. Modification du procédé selon la revendication 1, caractérisée en ce que l'on fait d'abord réagir les diamines et diisocyanates à utiliser dans le procédé ci-dessus mentionné en l'absence d'un catalyseur pour donner un uréediisocyanate de formule

$$OCN-R_4-NH-CO-\underset{\displaystyle |}{\overset{\displaystyle R_1}{N}}-R_2-\overset{\displaystyle R_3}{\underset{}{N}}-CO-NH-R_4-NCO$$

et ensuite on fait réagir cet uréediisocyanate en présence des catalyseurs mentionnés ci-dessus avec encore le diisocyanate de formule

$$R_4(NCO)_2$$

ou avec un autre diisocyanate de formule

$$R_5(NCO)_2$$

$R_1$, $R_2$, $R_3$ et $R_4$ ayant la signification indiquée ci-dessus, et $R_5$ correspondant à la définition de $R_4$, mais n'étant pas identique à $R_4$.

3. Polyisocyanates à groupes biuret et/ou à groupes polyuret supérieurs qui peuvent être obtenus selon les revendications 1 et 2.

4. Utilisation des polyisocyanates à groupes biuret et/ou à groupes polyuret supérieurs qui peuvent être obtenus selon les revendications 1 et 2, éventuellement sous forme bloquée par des agents de blocage des groupes isocyanate, comme composants de structure dans la préparation de matières plastiques de polyuréthannes selon le procédé de polyaddition des isocyanates.